(19) Europäisches Patentamt
European Patent Office
Office européen des brevets

(11) Publication number: **0 095 947**
**B1**

(12) **EUROPEAN PATENT SPECIFICATION**

(45) Date of publication of patent specification: **30.11.88**

(51) Int. Cl.⁴: **C 12 N 15/00,** C 12 N 1/20, C 12 P 21/00 // C12R1/125

(21) Application number: **83303195.8**

(22) Date of filing: **02.06.83**

(54) **Partition proficiency encoded DNA and plasmids incorporating the same for transforming gram-positive organisms.**

(30) Priority: **28.02.83 US 470576**
**02.06.82 US 384253**

(43) Date of publication of application:
**07.12.83 Bulletin 83/49**

(45) Publication of the grant of the patent:
**30.11.88 Bulletin 88/48**

(84) Designated Contracting States:
**AT BE CH DE FR GB IT LI LU NL SE**

(56) References cited:
**CHEMICAL ABSTRACTS, vol. 92, no. 17, 28th April 1980, page 275, no. 143073m, Columbus, Ohio, USA T. MIKI et al.: "Cloning of replication, incompatibility and stability functions of R plasmid NR1"**

**CELL, vol. 20, June 1980, pages 529-542 P.A. MEACOCK et al.: "Partitioning of bacterial plasmids during cell division: a cis-acting locus that accomplishes stable plasmid inheritance"**

(73) Proprietor: **CETUS CORPORATION**
**1400 Fifty-Third Street**
**Emeryville California 94608 (US)**

(72) Inventor: **Chang, Shing**
**118 Golden Rod Drive**
**Hercules California 94547 (US)**

(74) Representative: **Bizley, Richard Edward et al**
**BOULT, WADE & TENNANT 27 Furnival Street**
**London EC4A 1PQ (GB)**

(56) References cited:
**PLASMID, vol. 7, 1982, pages 163-179 R.W. SEELKE et al.: "Genetic studies of F plasmid maintenance genes involved in copy number control, incompatibility and partitioning"**

**PLASMID, vol. 4, 1980, pages 332-349 K. NORDSTRÖM et al.: "Partitioning of plasmid R1 in Escherichia coli. II. Incompatibility properties of the partitioning system"**

**J. Gen. Appl. Microbiol. 29, 1983, 345-354 Agric. Biol. Chem., 1978, 42 (10), 1841-1846**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 095 947 B1**

## Description

This invention relates generally to the field of molecular biology. In particular, the invention is specifically based upon a method for conferring long term stability to engineered recombinant plasmids which propagate in Gram-positive organisms.

The method of the invention may be used to construct novel engineered recombinant plasmids that are stably propagated within a growing population of Gram-positive organisms. A parental plasmid that can be used to construct the novel engineered recombinant plasmids of the invention is pLS11. Plasmid pLS11 is found in *Bacillus subtilis* strain IFO3022 which is on deposit at the Institute for Fermentation in Osaka, Japan. An example of a novel plasmid constructed according to the method of the present invention in *Bacillus subtilis* strain BD224 has been deposited in the American Type Culture Collection, Rockville, Maryland, 20852. This plasmid is identified as plasmid pOG2381. *Bacillus subtilis* strain BD224 harboring pOG2381 has been assigned ATCC Number 39038.

In order for autonomously replicating plasmids to be propagated stably within a growing bacterial cell population, several requirements must be satisfied. The rate of plasmid replication must be monitored by some cellular mechanism and adjusted to the growth rate of the culture. It has been suggested that this is probably accomplished through the action of a cytoplasmic agent which acts to regulate the frequency of replication initiation of the plasmid DNA molecule. The alleged cytoplasmic agent appears to be synthesized in proportion to the number of plasmid copies. *See* Pritchard, R., et al., *Symp. Soc. Gen. Microbiol.,* 19:263—297 (1968). In this way the plasmid is maintained at a constant concentration within the total cellular mass.

Stable maintenance of a plasmid in a growing cell population also requires that each of the daughter cells produced during cell division receive at least one copy of the plasmid from the parental cell. However, without a mechanism for the active partitioning of the plasmid, distribution of the plasmid molecules between the daughter cells would occur purely by random and a finite proportion of the cells would not inherit the plasmids. Since loss of naturally occurring plasmids is ordinarily not observed during growth of bacterial cell populations, it is now believed that most such plasmids undergo active partitioning at the time of cell division. *See* Meacock, P. and Cohen, S., *Cell* 20:529—542 (1980).

Stable maintenance of a plasmid in a growing cell population further requires structural stability of the plasmid DNA. When used in this manner, structural stability means faithful propagation of the plasmid DNA during replication.

The present invention relates to the component of stable plasmid propagation which involves active partitioning, i.e. partition proficiency. The invention is significant because, unfortunately, the "engineered" plasmids constructed by modern recombinant DNA techniques do not always undergo active partitioning at the time of host cell division. That is, many engineered recombinant plasmids are not partition proficient or are only weakly so. Lack of active partitioning is especially evident when genetic fragments taken from plasmids native to one type of organism are used in the construction of engineered recombinant plasmids capable of transforming another type of organism. For example, genetic fragments from plasmids native to *Staphylococcus aureus* bacteria have been used in the construction of synthetic plasmids capable of transforming *Bacillus subtilis* bacteria. Construction of such plasmids is described in European Patent Application Publication No. 0,036,259.

It has now been demonstrated that engineered recombinant plasmids and their progeny are not always stably maintained during cell population growth because they are not actively partitioned in cell divisions. Of course, lack of stable maintenance during cell population growth diminishes the industrial usefulness of engineered recombinant plasmids constructed to code for useful gene products. The maintenance of such structurally stable but not actively partitioned plasmids requires the presence in the growth medium of an antibiotic, or another type of selection pressure, against which the plasmid provides a selective advantage to cells harboring it. Stably maintaining plasmids in this way, e.g. with an antibiotic in the growth medium, is costly and inefficient.

The present invention, in one aspect, provides a *B. subtilis*-effective partition proficiency segment which when integrated into a recominant plasmid replicatable in *B. subtilis* confers partition proficiency to said recombinant plasmid in *B. subtilis* and which is obtainable from engineered recombinant *B. subtilis* plasmid pOG2381 (obtainable from ATCC 39,038), in the form of *MboI* or *Sau3A* fragments of approximately 1200 base pairs or *Hind*III—*Hae*III fragments of approximately 350 base pairs or *Hae*III—*Pst*I fragments of approximately 260 base pairs, or DNA fragments having homology to at least part of any of the aforesaid fragments and wherein the homologous fragments confer partition proficiency to recombinant plasmids in *B. subtilis.*

The invention also includes *B. subtilis* partition proficiency recombinant plasmids incorporating such segments and their manufacture and *B. subtilis* transformed thereby.

Another aspect of the invention is a method for obtaining *B. subtilis*-effective partition segments, which method comprises ligating DNA fragments as defined above into a compatible restriction enzyme site produced by enzymatically digesting a plasmid capable of transforming a *B. subtilis* host to produce engineered plasmids incorporating said DNA fragments, using the resulting engineered plasmids to transform cells of the host, growing the host cells after transformation and selecting for transformants, and, if desired, enzymatically digesting the resulting partition proficient plasmids and isolating the *B. subtilis* partition segments from the digest.

Another aspect of the invention provides a method for obtaining a *B. subtilis* effective partition segment as defined above which comprises

(a) digesting with a restriction enzyme a plasmid which occurs naturally in a *B. subtilis* species A or an engineered recombinant *B. subtilis* plasmid known to be partition proficient,

(b) randomly cloning the resulting DNA fragments obtained in step (a) into a plasmid capable of replication in a bacterial species B as well as *B. subtilis* C and bearing a selectable marker,

(c) transforming a population of organisms of species B with the recombinant plasmids produced in step (b),

(d) growing organisms of species B transformed as in step (c) under selection pressure corresponding to the selectable marker on the plasmids produced in step (b),

(e) harvesting plasmids from the organisms of species B grown as in step (d),

(f) transforming a population of *B. subtilis* with the plasmids harvested as in step (e),

(g) growing the population of transformed *B. subtilis* under the selection pressure to which the selectable marker on the plasmids corresponds for a number of doubling times sufficient to eliminate substantially all of the organisms not transformed by the plasmids in step (f),

(h) growing the population of organisms remaining after step (g) in the absence of the selection pressure corresponding to the selectable marker for a number of doubling times sufficient to increase the fraction of organisms in the population which bear partition proficient plasmids produced in step (b) to at least 0.5,

(i) isolating the plasmids from the population of organisms resulting after step (h),

(j) digesting the plasmids obtained in step (i) with a restriction enzyme, and

(k) isolating the Gram-positive partition segment from the fragments obtained in step (j).

The invention will now be further described and illustrated in the following description, taken in connection with the accompanying drawings, wherein:

Figure 1 shows restriction enzyme maps that detail the construction of plasmid pOG2326.

Figure 2 shows a restriction enzyme map that details construction of plasmid pOG2381 (ATCC 39038).

Figure 3 is a graph that illustrates partition proficiency of plasmid pOG2381 which is stably maintained, and pOG2326 which is not stably maintained, in populations of *Bacillus subtilis* strain BD224 growing in the absence of the antibiotic chloramphenicol. The stable maintenance of pOG2381 demonstrates its partition proficiency. The lack of stable maintenance of pOG2326 is due to its lack of partition proficiency.

Figure 4 shows a restriction enzyme map that details the construction of plasmid pDH5060.

Figure 5 shows a restriction enzyme map of plasmid pSYC706.

Figure 6 shows the nucleotide sequence of a *Hae*III to *Hind*III DNA fragment of approximately 350 base pairs contained within the 1.2kb *Mbo*I Gram-positive partition fragment.

Figure 7 shows a restriction enzyme map of a *Hae*III to *Hind*III fragment of approximately 350 base pairs contained within the 1.2kb *Mbo*I Gram-positive partition fragment.

Very generally, the invention discloses a method useful in the production of engineered recombinant plasmids that will be partition proficient and stably transmitted during Gram-positive host cell divisions and stably maintained during growth of host cell populations. Engineered recombinant plasmids produced by the method of the invention are capable of replication in Gram-positive organisms and include a partition segment, *par*$^+$, which confers the partition proficiency in host cell divisions and is required for stable maintenance of the engineered recombinant plasmids within growing Gram-positive host cell populations. The engineered recombinant plasmids can include DNA coding for a desired protein or other gene products.

As used herein, engineered recombinant plasmid means a plasmid constructed using techniques of genetic engineering.

Techniques of genetic engineering mean techniques which lead to the formation of new combinations of heritable material by the insertion of nucleic acid molecules, produced or derived by whatever means outside the cell, into a bacterial plasmid or other vector system so as to allow their incorporation into a host organism in which they do not naturally occur at high frequency but in which they are capable of replication.

A Gram-positive organism means a Gram--positive bacterium.

A transformable host means a Gram-positive host organism capable of accepting plasmid DNA from Gram-positive *par*$^+$ recombinant plasmids. Gram-positive transformable host organisms are thus capable of being transformed by Gram-positive *par*$^+$ recombinant plasmids.

A compatible host organism means a transformable Gram-positive host organism capable of allowing replication of the *par*$^+$ recombinant plasmid DNA. Said compatible host organism will also allow expression of the *par*$^+$ phenotype, in addition to at least one other gene carried the Gram-positive *par*$^+$ recombinant plasmids.

Progeny means offspring or daughter cells produced during cell division where the offspring or daughter cells are substantially equivalent to the parental cell.

Partitioning means the process, random or active, whereby copies of a plasmid are distributed or transmitted to daughter cells during host cell division.

Active partitioning means partitioning of plasmids in a partition proficient plasmid-host cell system.

Partition proficiency is a phenotypical trait of a plasmid in a cell such that copies of the plasmid are partitioned in cell division by other than purely random processes. Partition proficiency in a plasmid-Gram-positive host cell system requires the presence of a Gram-positive partition segment on the plasmids of such a system.

A Gram-positive partition segment comprises a sequence of DNA nucleotides, herein defined as a partition proficiency gene (*par*$^+$), which must be on a plasmid for partition proficiency.

If a plasmid lacks the partition proficiency gene, the plasmid will be unevenly partitioned during cell division. Unless the plasmid confers a selective advantage on cells harboring it, its prevalence in the host cells may decrease at a substantial rate after several generations of cell divisions. Thus production of useful gene products from genes carried on the plasmids may diminish or stop entirely.

The *par*$^+$ segment can be derived from naturally occurring Gram-positive plasmids. Examples of such plasmids have been described by Tanaka, T., et al., *J. Bacteriol.,* (129:1487—1494 (1977). The *par*$^+$ segment can also be obtained from engineered recombinant plasmid pOG2381 (ATCC 39038).

An engineered recombinant *par*$^+$ plasmid of the invention will have a stability in growing, Gram-positive host cell populations that is greater than that of an engineered recombinant plasmid that is structurally the same but for lack of the partition segment. *Par*$^+$ engineered recombinant plasmids of this invention may be obtained having stability as high as 100% after at least 100 generations of Gram-positive host cell growth. As used herein, one generation means a doubling of host cell number.

In practicing the method of the invention, naturally occurring Gram-positive plasmids that contain the *par*$^+$ function are cleaved with restriction enzymes to create linear DNA segments having ligatible termini. The partition proficiency gene will be contained on some of the linear DNA fragment segments (Gram-positive partition segments). The partition proficiency gene can also be obtained from engineered recombinant plasmid pOG2381 (ATCC 39038). Plasmid pOG2381 may be cleaved with restriction enzymes to yield a fragment which contains the partition proficient gene. Cleavage with restriction enzymes *Mbo*I or *Sau*3A yields a large fragment of about 1200 bp (1.2kb) which includes the partition proficient gene. Cleavage with restriction enzymes *Hae*III and *Hind*III yields a smaller fragment of about 350 base pairs which also includes the partition proficient gene. Cleavage of pOG2381 with restriction enzymes *Pst*I and *Hae*III yields a still smaller fragment of about 260 bp which includes the partition proficient gene. Recombinant plasmids capable of replication in Gram-positive host organisms are also cleaved with restriction enzymes to yield linear DNA having termini ligatible with the termini of Gram-positive partition gene segments. The termini are bound together to form novel recombinant plasmids used to transform transformable and compatible Gram--positive host organisms. After transformation, the cells are grown and selection is made for partition proficient (*par*$^\pm$) plasmids. Transformants carrying the *par*$^+$ plasmids are harvested and used in applications where long term growth or fermentation for the production of protein or other gene products is desired.

The preferred source of the partition proficiency gene, the 260 bp *Hae*III—*Pst*I fragment, the 350 bp *Hae*III—*Hind*III fragment, or the 1.2kb *Mbo*I or *Sau*3A fragment from plasmid pOG2381 (ATCC 39038) (also described herein as Gram-positive *par*$^\pm$ fragments) may be ligated into a compatible restriction enzyme site on an engineered recombinant plasmid capable of transforming Gram-positive bacteria. Especially preferred as engineered recombinant plasmids are the recombinant plasmids disclosed in European Patent Application Publication Number 0,036,259. The novel engineered recombinant plasmids carrying the Gram-positive *par*$^+$ fragment are used to transform transformable and compatible Gram-positive host cells. Especially preferred is *Bacillus subtilis* strain BD224 (trpC2 thr-5 recE4). Transformants are selected and then inoculated into broth without antibiotics and grown for about 50 generations to allow the unstable partition deficient, *par*$^-$, clones to segregate. Transformants carrying the *par*$^+$ plasmids are harvested and used in the production of desired gene products.

The following Examples illustrate ways in which the invention may be employed. They are included only for purposes of illustration and are not intended to limit the scope of the invention.

Example 1

Construction of engineered recombinant plasmids containing a Gram-positive partition fragment, *par*$^+$, from a *Bacillus subtilis* plasmid.

Plasmid pLS11 was isolated and purified from *Bacillus subtilis* strain IFO3022. *See* Tanaka, T., et al., *J. Bacteriol.,* 129:1487—1494, (1977). Two micrograms of pLS11 DNA were digested with 2 units of restriction enzyme *Mbo*I, (New England Biolabs, *supra*), in 50 microliters of 6mM Tris-HCl, pH 7.5, 6mM $MgCl_2$, 50mM NaCl, at 37°C for 30 minutes.

Plasmid DNA from pLS11 was ligated with DNA originating from the engineered recombinant plasmids described in European Patent Application Publication Number 0,036,259. Specifically, DNA from pLS11 was ligated with DNA from plasmid pOG2326.

Plasmid pOG2326, which replicates in both *Bacillus subtilis* and *Escherichia coli* was constructed from *Bacillus subtilis* plasmid pOG1196 (ATCC 31776) and *Escherichia coli* plasmid pBR322 (ATCC 37017). *See* Fig. 1. In constructing pOG2326, 3 micrograms of DNA from plasmid pOG1196 was digested with *Mbo*I enzyme under the same conditions described above for pLS11 DNA. The digested DNA was ligated with 2 units of T4-DNA ligase in 20 microliters of 66mM Tris-HCl, pH 7.6, 6.6mM $MgCl_2$, 10mM DTT, 0.1mM ATP, at 14°C for 18 hours. After transformation of *Bacillus subtilis* BD224 with the ligated DNA, a plasmid-carrying, chloramphenicol-resistant transformant was characterized further. The plasmid found in the transformant was designated pDH1086; it had the same structure as pOG1196 except that a small *Mbo*I fragment has been deleted. *See* Fig. 1. Two micrograms each of plasmid pHD1086 and *Escherichia coli* plasmid pBR322, were digested with *PVU*II endonuclease under the same conditions described above for *Mbo*I. The resulting fragments were ligated with T4-DNA ligase using procedures described *supra* for this ligase. The ligated DNA was used to transform *Escherichia coli* strain CS412 (an hsdR$^-$ derivative of C600 strain); transformants resistant to chloramphenicol were selected. The plasmid from one transformant, designated pOG2326, was characterized further. It has the structure shown in Fig. 1. The low stability of plasmid pOG2326 was shown by the fact that, after 10 generations in broth without antibiotics, less than 0.002% of the cells in a population still harbored the plasmid, when assayed using chloramphenicol (Cm) resistance as described below. These data are shown in Fig. 3.

In constructing plasmid pOG2381, 0.5 micrograms of DNA from plasmid pOG2326 was digested with *Bam*H1 enzyme; then 2 micrograms of the *Mbo*I fragments of pLS11 were added. The pOG2326 fragments and the pLS11 fragments were ligated with T4-DNA ligase using procedures described *supra* for this ligase. The ligated DNA was used to transform *Escherichia coli* strain CS412 (an *hsd*R$^-$ derivative of C600 strain). Those skilled in the art will recognize that any other *hsd*R$^-$ strain of *Escherichia coli* could have been used in this transformation.

Since insertion into the *BAM*H1 site inactivates the tetracycline resistant (*tet*) gene, *Escherichia coli* transformants harboring chimeric plasmids containing cloned pLS11 sequences showed ampicillin-resistant, tetracycline-sensitive (Ap$^R$Tc$^S$) phenotype. One hundred Ap$^R$ transformants were tested; 47% of Ap$^R$ clones carried recombinant plasmids derived from pOG2326. Plasmid DNA was made from a mixed culture of about one hundred ampicillin-resistant, tetracycline-sensitive (Ap$^R$Tc$^S$) transformants. The plasmid DNA was then transformed into *Bacillus subtilis* strain BD224 (trpC2 thr-5 recE4). Transformants were selected initially on Cm-containing plates. Following this selection, about one thousand of the selected clones were innoculated into broth without antibiotics and grown for about 50 generations. This allowed unstable partition deficient, *par*$^-$, clones to segregate. This culture was then grown overnight in Cm-containing broth to enrich for the *par*$^+$ clones. After two more cycles of this type of segregation-enrichment treatment, eight individual clmces were isolated to test for *par*$^+$ phenotype. They were all 100% stable. Stability was tested by growing transformed BD224 strain in broth without antibiotics for 100 generations. One hundred or more colonies from each culture were grown up on plate and tested individually for resistance to Cm. One plasmid, pOG2381, ATCC Number 39038, isolated from these clones was characterized further. It contains an *Mbo*I insert of about 1.2kb at the *Bam*H1 site of pOG2326. Upon transformation into BD224, it again showed *par*$^+$ phenotype demonstrating that the phenotype is associated with sequences on the plasmid.

To further verify that the *par*$^+$ fragment is located within a 1.2 kb *Mbo*I fragment from pOG2381, the plasmid was digested with *Sau*3A. Both *Sau*3A and *Mbo*I recognize the same DNA sequences:

5'...GATC...3'
3'...CTAG...5'.

After purification, the 1.2kb *Sau*3A fragment was cloned into the *Bam*H1 site of pOG2326. The resulting construction was *par*$^+$ and showed a structure identical to pOG2381. This demonstrates that the partition proficient gene is located within a *Mbo*I or *Sau*3A 1.2kb fragment from pOG2381. This fragment carries the Gram--positive partition segment.

Example 2

Demonstration that *Bacillus subtilis par*$^+$ does not function in *Escherichia coli.*

It has been documented that a partition function is required for stable maintenance of *Escherichia coli* plasmids in *Escherichia coli. See* Meacock, P., and Cohen, S., *Cell,* 20:529—542 (1980). Since some cloned *Bacillus subtilis* genes are functional in *Escherichia coli,* the cloned *Bacillus subtilis par*$^+$ plasmids were tested in *Escherichia coli* strain CS412 using methods described in Example 1. When tested for partition, both plasmids pOG2326 and pOG2381 were phenotypically *par*$^-$ in *Escherichia coli.* After one hundred generations, grown in broth without antibiotics, no Ap$^R$ clone was detected among the one hundred clones tested.

Example 3

Construction of engineered recombinant plasmids containing a Gram-positive partition fragment, *par*$^+$*,* from a *Bacillus subtilis* plasmid, and a heterologous gene coding for a secreted protein product.

To ascertain whether the presence of the Gram-positive partition fragment interferes with the expression and secretion of protein product in Gram-positive bacteria, a plasmid was constructed that carries both the Gram-positive *par*$^+$ fragment and a heterologous gene coding for a protein known to be expressed and secreted in *Bacillus subtilis.* The plasmid thus constructed has been designated pOG2428. In constructing plasmid pOG2428, a hybrid plasmid was made first that carried sequences from *Escherichia coli* plasmid pBR322 (ATCC 37017) and *Bacillus subtilis* plasmid pOG1196 (ATCC 31776). *Escherichia coli* plasmid pBR322 has been described by Sutcliffe, J. G., *Nucleic Acids Res.* 5:2721—2728 (1978), and *Cold Spring Harbor Symp. Quant. Biol.* 43:77—90 (1979). *Bacillus subtilis* plasmid pOG1196 has been described by Gray, O., and

Chang, S., *J. Bacteriol.* 145:422—428 (1981). Plasmids pBR322 and pOG1196 were each digested with the *PVU*II restriction enzyme and then ligated and transformed into CS412 strain of *Eschericia coli*; chloramphenicol resistant transformants were selected. A plasmid from one transformant, plasmid pDH5060 was utilized further. A restriction enzyme map of pDH5060 is shown in Fig. 4. The conditions used for digestion and ligation of the plasmid fractions, as well as transformation of the host, and selection are standard procedures well known to those skilled in the art of recombinant DNA. These and other molecular cloning techniques utilized in practicing the present invention can be found in Maniatis, T., et al., *Molecular Cloning: A Laboratory Manual*, Cold Spring Harbor Laboratory (1982).

Plasmid pDH5060 was digested with restriction enzymes *Eco*RI and *Bam*HI. The "large plasmid fragment" produced by this enzyme digestion was purified from an agarose gel as described by Maniatis, T., et al, *supra*.

To provide a gene coding for a heterologous protein known to be expressed and secreted in *Bacillus subtilis*, an *Eco*RI-*Bam*III-enzyme generated fragment containing the *Bacillus lichenformis pen*P gene was purified from plasmid pSYC310-2. This plasmid has been described by McLaughlin, J., et al., *Nucleic Acid Res.* 10:3905—3919 (1982). Those skilled in the art will realize the *Bacillus licheniformis pen*P could have been obtained from other sources. *See* Gray, O., and Chang, S., *J. Bacteriol.* 145:422—428 (1981). The purified "large fragment" from plasmid pDH5060 and the fragment containing the *Bacillus licheniformis pen*P gene from plasmid pSYC310-2 where ligated and transformed into *Escherichia coli* strain CS412; transformants resistant to ampicillin were selected. A plasmid from one transformant, designated pSYC189 was utilized further.

In the plasmid, pSYC189, a small deletion was made in the *bla* gene which originated from retained portion of the plasmid pBR322. *See* Sutcliffe, J. G., *Cold Spring Harbor Symp. Quant. Biol.* 43:77—90 (1979). The *bla* gene encodes beta-latamase protein. To generate the small deletion in the beta-lactamase gene, the plasmid was digested at the *Pvu*I site in the *bla* gene. The resulting *Pvu*I digested plasmid DNA was treated with exonuclease BAL-31 using conditions well-known to those skilled in the art. Such conditions are described in Maniatis, T., et al., *supra*. The BAL-31 treated fragment was recircularized by ligation. This digestion deleted about 120 base pairs from from the wild-type sequence of beta-lactamase gene, resulting in a plasmid designated pSYC274.

At the 3'-end of the penicillinase gene in plasmid pSYC274 there is a restriction site for *Bcl*I comprising of the recognition sequence: TGATCA. The location of this restriction enzyme site can be found in the sequence reported by Neugebauer, K., et al., *Nucleic Acids Research* 9:2577—2588 (1981). The *Bcl*I site is at the non-translated region near the very end of the penicillinase gene. The *Bcl*I site at the end of penicillinase gene is a unique site for this enzyme on plasmid pSYC274.

Digestion with *Bcl*I leaves GATC protruding ends which may be ligated with GATC protruding ends generated when DNA is digested with *Mbo*I. The 1.2kb partition fragment from pOG2381 was excised by digesting the plasmid with *Mbo*I. The 1.2kb *Mbo*I partition fragment thus obtained was cloned into the *Bcl*I site of pSYC274. Ligation of these two plasmid fragments generated a hybrid plasmid designated pOG2428. This plasmid contains the penicillinase gene from *Bacillus licheniformis*, which codes for a product secreted by *Bacillus subtilis* transformed hosts; the plasmid also contains the Gram-positive partition fragment.

Example 4

Demonstration that the presence of a Gram-positive *par*$^+$ fragment does not interfere with the expression or secretion or a heterologous protein produced by transformed *Bacillus subtilis* bacteria.

Gray and Chang have shown that the cloned beta-lactamase, hereinafter referred to as penicillinase or *pen*P, gene from *Bacillus licheniformis* is functionally expressed in *Bacillus subtilis*. *See* Gray, O. and Chang, S., *J. Bacteriol.* 145:422—428 (1981). The heterologous penicillinase molecules produced in transformed *Bacillus subtilis* cells are translocated across the limiting membrane and subsequently processed proteolytically to form the penicillinase exoenzyme.

To determine whether the presence of the Gram-positive *par*$^+$ interfered with the expression or secretion of *Bacillus licheniformis* penicillinase by transformed *Bacillus subtilis* host organisms, plasmid pOG2428 was transformed into *Bacillus subtilis* strain BD224. When penicillinase activity in solution was determined spectrophotometrically using nitrocefin (chromogenic cephalosporin 87/312, available from Glaxo Research Limited) by the method of O'Callaghan, C., et al., results showed that there where no decrease in detectable enzyme activity due to the presence of the *par*$^+$ fragment on pOG2428. *See* O'Callaghan C., et al. *Antimicrob. Agents Chemother* 1:283—288 (1972).

Example 5

The Gram-positive partition function is located within a fragment of about 350 base pairs contained within the larger 1.2kb *Mbo*I fragment.

The 1.2kb *Mbo*I fragment contains a *Hind*III restriction site. *See* Fig. 2. Mapping with restriction enzymes revealed a *Hae*III site located about 350 base pairs away from the *Hind*III in the 1.2 kb *Mbo*I fragment. Double digestion of plasmid pOG2381 with restriction enzymes *Hae*III and *Hind*III made it possible to purify this 350 base pair fragment from an agarose gel. The purified 350 base pair fragment was cloned into an engineered recombinant plasmid to determine whether the Gram-positive partition function was located within this smaller 350 bp fragment.

Plasmid pDH5060 was treated with *Hind*III enzyme so that it would only partially cut at one of the two *Hind*III sites located on that plasmid. The locations of the *Hind*III sites in plasmid pDH5060 are illustrated in Fig. 4. The partially digested pDH5060 DNA preparation was treated with *Escherichia coli* DNA polymerase I Klenow fragment as described by Maniatis, T., et al., *supra.* After the *Hind*III generated protruding ends had been filled in with the *Escherichia coli* DNA polymerase I Klenow fragment, they where ligated using standard methods. The ligated DNA was transformed into *Escherichia coli* strain CS412 competent cells. The transformed *Escherichia coli* cells where screened and a clone was identified which carried a plasmid that has lost one of the two *Hind*III sites. This plasmid is designated pLP1201. It retains the *Hind*III site located in the tetracycline gene but has lost the *Hind*III site that originated from the bacillus replicon.

Plasmid pLP1201 was digested with *Hind*III and *Bam*HI restriction enzymes. *Bam*HI was used first. Once the *Bam*HI protruding ends had been filled in with *Escherichia coli* DNA polymerase I Klenow fragment, the plasmid was digested with *Hind*III. A partition fragment of approximately 350 base pairs obtained by the method described above was added to this DNA preparation. The DNA mixture was ligated, and then used to transform *Escherichia coli* strain CS412 competent cells. The transformants were screened by isolating and analyzing the DNA structures for the presence of the partition fragment. When a repaired *Bam*HI site is ligated to a *Hae*III site, a *Bam*HI site will be regenerated. Thus it was possible to first simply digest plasmid DNA prepared from the transformants, and then identify the desirable transformant which had the *Hind*III to *Bam*HI fragment of appropriate size. A transformant carrying a plasmid designated pSYC706 was deemed to have the *Hind*III to *Bam*HI fragment of appropriate size. The structure of pSYC706 is shown in Fig. 5. When *Bacillus subtilis* strain 224 carrying plasmid pSYC706 was tested for partition proficiency, it was shown to have a phenotype identical to plasmid pOG2381. *Bacillus subtilis* cells BD224 harboring plasmid pSYC706 did not spontaneously lose the plasmid as did *Bacillus subtilis* cells BD224 carrying the parental plasmid. This experiment clearly demonstrated that the approximate 350 base pair sequence located between the *Hind*III site and the *Hae*III site in the 1.2kb *Mbo*I fragment contains the Gram-positive partition proficiency function.

Example 6

The Gram-positive partition function is located within a fragment of about 260 base pairs contained within the 350 bp *Hind*III—*Hae*III fragment.

The nucleotide sequence in the fragment of approximately 350 base pairs located between the *Hind*III and *Hae*III sites in the 1.2kb *Mbo*I Gram-positive partition fragment was determined. The sequence of this fragment is shown in Fig. 6.

To determine whether the Gram-positive partition proficiency function is located between the *Hae*III and *Pst*I sites within the 1.2kb *Mbo*I fragment, a fragment of approximately 260 base pairs fragment was isolated by digesting plasmid pOG2381 with restriction enzymes *Hae*III and *PSt*I. The 260 base pair was purified on an agarose gel. Plasmid pDH5060 was digested with restriction enzyme *Eco*RI; the 5′ protruding ends were filled in by DNA polymerase I Klenow fragment as described by Maniatis, T., et al., *supra.* The preparation was further digested with *Pst*I enzyme to generate 2 fragments. The purified *Hae*III—*Pst*I fragment of approximately 260 base pairs was added at equal molar ratio to the preparation described above. It was then ligated with the digested pDH5060 fragments. The ligated DNA was used to transform *Escherichia coli* strain CS412. A tetracycline-resistant ampicillin-sensitive transformant was characterized further. It carries a plasmid designated pSYC730 which has the 260 base pair Gram-positive *par*$^+$ fragment cloned in place of the small *Eco*RI to *Pst*I fragment on pDH5060. Plasmid pSYC730 was transformed into *Bacillus subtilis* strain BD244 and then tested for partition proficiency. The results of partition proficiency test revealed that after 20 generations of growth in broth without chloramphenicol, *Bacillus subtilis* strain BD224 carrying plasmid pDH5060 was unstable. The same strain BD224 carrying *par*$^+$ plasmid pSYC730 was stable. Thus it is concluded that the Gram-positive *par*$^+$ proficiency is located within the approximate 260 base pair region flanked by the *Hae*III and *Pst*I sites as shown in Fig. 6.

Example 7

The DNA sequence of the Gram-positive *par*$^+$ proficiency function.

The sequence of the DNA from pSYC706 that contains the partition function was determined using the methods developed by Gilbert, W., and Maxam, A., in *Methods in Enzymology* 65:499—560 (edited by Grossman, L., and Moldave, K. J.) Academic Press, (1980). Fig. 6 shows the nucleotide sequence of this DNA. There is some ambiguity in the region around positions 60 to 70 due to compression which is probably caused by the presence of the GC-rich region adjacent to it (located between positions 20 to 65). However, many restriction enzyme sites found in this fragment, as predicted by sequence analysis, are also found by actual restriction enzyme digestion.

Example 8

The Gram-positive *par*$^+$ recombinant plasmids of the present invention show improved stability in Gram-positive *rec*E$^+$ transformed hosts.

It has been shown in the previous Examples that the Gram-positive *par*$^+$ recombinant plasmids of the present invention show improved stability when transformed into a *rec*E⁻ strain of *Bacillus subtilis* (BD224). As used herein, improved stability means that the recombinant plasmids with the Gram-positive *par*⁺ fragment are more stable than are identical plasmids that do not contain the Gram-positive *par*⁺ fragment. To determine whether the *par*⁺ plasmids show improved stability in a *rec*E⁺ strain of *Bacillus subtilis,* the Gram-positive *par*⁺ recombinant plasmids were used to transform a *rec*E⁺ strain of *Bacillus subtilis* (BD170). Except for being *rec*E⁺, *Bacillus subtilis* strain BD170 is identical to strain BD224.

When *Bacillus subtilis* strain BD170 was transformed with plasmid pOG2381, it was 99% stable, i.e., pOG2381 was present in 99% of the transformed host cells, after 15 generations. When strain BD170 was transformed with pOG2428, it was 100% stable after 14 generations. Transformation techniques were identical to those described in the previous examples.

Example 9

The Gram-positive *par*⁺ fragment of the present invention shows an orientation dependency.

The partition fragment of the present invention can be inserted into the recombinant plasmids in two possible orientations. Only one orientation shows the partition phenotype. The reason for the orientation dependency of the *par*⁺ fragment is unclear.

It may be seen therefore that the invention provides compositions and methods useful for the production of engineered recombinant plasmids that include a partition segment which enhances the stability of the plasmids in Gram-positive host cell populations, especially growing Gram-positive host cell populations. The invention also provides *par*⁺ fragments as well as *par*⁺-engineered recombinant plasmids, i.e., engineered recombinant plasmids which include a Gram-positive partition segment. It further provides, engineered Gram-positive *par*⁺ recombinant plasmids which are substantially more partition stable than are identical plasmids that do not contain such a *par*⁺ partition fragment. Further, it provides Gram-positive organisms transformed with engineered *par*± recombinant plasmids. The engineered *par*± recombinant plasmids, and organisms transformed with them, are especially useful in industrial applications where long term growth or fermentation, e.g. a continuous fermentation process, is desired for the production of protein products.

Various modifications within the broad scope of the invention in addition to those shown and described herein will become apparent to those skilled in the art from the foregoing description.

References


1. Gilbert, W., and Maxam, A., in *Method in Enzymology* 65:499—560 (edited by Grossman, L., and Moldave, K. J.) Academic Press, (1980).
2. Gray,. O. and Chang, S., *J. Bacteriol.* 145:422—428 (1981).
3. Maniatis, T., Fritsch, E. F., and Sambrook, J. *Molecular Cloning: A Laboratory Manual,* Cold Spring Harbor Laboratory (1982).
4. McLaughlin, J., Chang, S. Y., and Chang, S., *Nucleic Acid Res.* 10:3905—3919 (1982).
5. Meacock, P. and Cohen, S., *Cell* 20:529—542 (1980).
6. Neugebauer, K., Sprengel, R., and Schaller, H., *Nucleic Acids Research* 9:2577—2588 (1981).
7. O'Callaghan, C., Morris, A., Kirby, S. M., and Shingler, A. H., *Antimicrob. Agents Chemother* 1:283—288 (1972).
8. Pritchard, R. H., Barth, P. T. and Collins J., *Symp. Soc. Gen. Microbiol.* 19:263—297 (1968).
9. Sutcliffe, J. G., *Nucleic Acids Res.* 5:2721—2728 (1978) and *Cold Spring Harbor Symp. Quant. Biol.* 43:77—90 (1979).
10. Tanaka, T., Kuroda, M., and Sakaguchi, K., *J. Bacteriol.* 129:1487—1494 (1977).


**Claims**

1. A *B. subtilis*-effective partition proficiency segment which when integrated into a recominant plasmid replicatable in *B. subtilis* confers partition proficiency to said recombinant plasmid in *B. subtilis* and which is obtainable from engineered recombinant *B. subtilis* plasmid pOG2381 (obtainable from ATCC 39,038), in the form of *Mbo*I or *Sau*3A fragments of approximately 1200 base pairs or *Hind*III—*Hae*III fragments of approximately 350 base pairs or *Hae*III—*Pst*I fragments of approximately 260 base pairs, or DNA fragments having homology to at least part of any of the aforesaid fragments and wherein the homologous fragments confer partition proficiency to recombinant plasmids in *B. subtilis*.

2. A partition proficiency segment comprising at least a portion of the nucleotide sequence of Figure 6 herein, or a DNA fragment having homology thereto, and wherein said segment or fragment is capable of conferring partition proficiency to recombinant plasmids in *B. subtilis.*

3. A *B. subtilis* partition proficiency recombinant plasmid which when transformed into *B. subtilis* is partition proficient therein and incorporates a partition segment or fragment as claimed in claim 1 or claim 2.

4. Plasmid pOG2381 (obtainable from ATCC 39038).

5. *B. subtilis* transformed by a recombinant plasmid as claimed in claim 3 or 4.

6. *B. subtilis* as claimed in claim 5 which is a transformed *Bacillus subtilus (rec*E⁻) organism.

7. *B. subtilis* as claimed in claim 6 which is *Bacillus subtilis* strain BD224 (obtainable from ATCC 39038).

8. A method for obtaining *B. subtilis*-effective partition segments, which method comprises ligating DNA fragments as defined in claim 1 or claim 2 into a compatible restriction enzyme site produced by enzymatically digesting a plasmid capable of transforming a *B. subtilis* host to produce engineered plasmids incorporating said DNA fragments, using the resulting engineered plasmids to transform cells of the host, growing the host cells after transformation and selecting for transformants, and, if desired, enzymatically digesting the resulting partition proficient plasmids and isolating the *B. subtilis* partition segments from the digest.

9. A method as claimed in claim 8 wherein the plasmid capable of transforming a *B. subtilis* host is plasmid pOG1196 (obtainable from ATCC 31776).

10. A method as claimed in claim 8 or claim 9, wherein the strain of *Bacillus subtilis* is *Bacillus subtilis* strain BD244 (obtainable from ATCC 39038).

11. A method for obtainaing a *B. subtilis* effective partition segment as defined in claim 1 or claim 2 which comprises

(a) digesting with a restriction enzyme a plasmid which occurs naturally in a *B. subtilis* species A or an engineered recombinant *B. subtilis* plasmid known to be partition proficient,

(b) randomly cloning the resulting DNA fragments obtained in step (a) into a plasmid capable of replication in a bacterial species B as well as *B. subtilis* C and bearing a selectable marker,

(c) transforming a population of organisms of species B with the recombinant plasmids produced in step (b),

(d) growing organisms of species B transformed as in step (c) under selection pressure corresponding to the selectable marker on the plasmids produced in step (b),

(e) harvesting plasmids from the organisms of species B grown as in step (d),

(f), transforming a population of *B. subtilis* with the plasmids harvested as in step (e),

(g) growing the population of transformed *B. subtilis* under the selection pressure to which the selectable marker on the plasmids corresponds for a number of doubling times sufficient to eliminate substantially all of the organisms not transformed by the plasmids in step (f),

(h) growing the population of organisms remaining after step (g) in the absence of the selection pressure corresponding to the selectable marker for a number of doubling times sufficient to increase the fraction of organisms in the population which bear partition proficient plasmids produced in step (b) to at least 0.5,

(i) isolating the plasmids from the population of organisms resulting after step (h),

(j) digesting the plasmids obtained in step (i) with a restriction enzyme, and

(k) isolating the Gram-positive partition segment from the fragments obtained in step (j).

12. A method for creating engineered recombinant plasmids that will be partition proficient during *B. subtilis* cell divisions comprising ligating *B. subtilis* partition segments or fragments as defined in claim 1 or claim 2 into recombinant plasmids.

13. A method as claimed in claim 11 or claim 12, wherein the engineered recombinant plasmid into which the *B. subtilis* partition segments or fragments are ligated is plasmid pOG1196 (obtainable from ATCC 31776).

14. A method for obtaining a population of *B. subtilis* carrying as part of their genotype phenotypically expressible partition proficient plasmids, which method comprises transforming *B. subtilis* with a plasmid as claimed in claim 3 or claim 4, followed by (if appropriate), or alternatively, in a population of *B. subtilis* harboring recombinant plasmids which carry a selectable marker, increasing the fraction of the organisms wherein the plasmids are partition proficient by growing the organisms in the absence of the selection pressure corresponding to the selectable marker.

15. A method as claimed in claim 14, wherein the selectable marker is resistant to antibiotic.

16. A method as claimed in claim 15, wherein the antibiotic is chloramphenicol.

17. A method as claimed in any one of claims 14 to 16, wherein the *B. subtilis* is *Bacillus subtilis* strain BD224 (obtainable from ATCC 39038).

18. A method as claimed in any one of claims 14 to 17, wherein the growth is continued for a number doubling times sufficient to increase the fraction of partition proficient plasmid-bearing organisms to more than about 0.90.

19. A process for preparing a protein product which comprises cultivating an organism as claimed in any one of claims 5 to 7 or a population of *B. subtilis* as defined in claim 14 and as obtained from a method as claimed in claim 14, wherein the partition proficient engineered recombinant plasmid carries genetic material encoded for the production of said protein product, and allowing the organism phenotypically to express its genotype including said plasmid.

**Patentansprüche**

1. Segment für die kontrollierte Weitergabe in in B. subtilis, das nach Integration in eine in B. subtilis replikationsfähiges, rekombinantes Plasmid dem Plasmid die Fähigkeit für die kontrollierte Weitergabe in B. subtilis verleight und das aus dem konstruierten, rekombinanten B. subtilis-Plasmid pOG2381 (erhältlich unter ATCC 39,038) in Form eines etwa 1200 Basenpaare großen Mbol- oder Sau3A-Fragments, eines etwa 350 Basenpaare großen HindIII-HaeIII-Fragments oder eines etwa 260 Basenpaare großen HaeIII-PstI-Fragments oder aus DNA-Fragmenten erhältich ist, die homologe Bereiche zumindest zu einem Teil eines der vorstehend genannten Fragmente enthalten, wobei die homologen Bereiche rekombinanten Plasmiden die Fähigkait für die kontrollierte Weitergabe in B. subtilis verleihen.

2. Segment für die kontrollierte Weitergabe, das zumindest einen Teil der Nucleotid-Sequenz von Fig. 6 umfaßt, oder ein DNA-Fragment mit einem homologen Bereich dazu, wobei das Segment oder Fragment in der Lage ist, rekombinanten Plasmiden die Fähigkeit für die kontrollierte Weitergabe in B. subtilis zu verleihen.

3. Rekombinantes Plasmid für die kontrollierte Weitergabe in B. subtilis, das nach Tranformation in B, subtilis kontrolliert weitergegeben wird und ein Segment oder Fragment für die kontrollierte Weitergabe nach Anspruch 1 oder 2 enthält.

4. Plasmid pOG2381 (erhältlich unter ATCC 39,038).

5. B. subtilis transformiert mit einem rekombinanten Plasmid nach Anspruch 3 oder 4.

6. B. subtilis nach Anspruch 5, nämlich ein transformierter Bacillus subtilis ($recE^-$) Organismus.

7. B. subtilis nach Anspruch 6, nämlich der Bacillus subtilis-Stamm BD224 (erhältlich unter ATC 39,038).

8. Verfahren zur Gewinnung von Segmenten mit kontrollierter Weitergabe in B. subtilis, gekennzeichnet durch die folgenden Schritte:

—Inserierung von DNA-Fragmenten nach Anspruch 1 oder 2 in eine geeignete, durch enzymatische Spaltung geöffnete Restriktionsstelle eines zur Transformation eines B. subtilis-Wirts geeigneten Plasmids zur Herstellung von konstruierten Plasmiden, welche diese DNA-Fragmente enthalten;

—Verwendung der erhaltenen konstruierten Plasmide zur Transformation von Wirtszellen;

—Kultivierung der Wirtszellen nach Transformation und Selektion auf Transformanten; und

—gegebenenfalls enzymatischz Spaltung der erhaltenen Plasmide für die kontrollierte Weitergabe und Isolierung der durch die Verdauung erhaltenen Segmente für die kontrollierte Weitergabe in B. subtilis.

9. Verfahren nach Anspruch 8, wobei das zur Transformation eines B. subtilis-Wirts geeignete Plasmid das Plasmid pOG1196 (erhältlich unter ATCC 31,776) ist.

10. Verfahren nach Anspruch 8 oder 9, wobei der Bacillus subtilis-Stamm der Stamm BD244 (erhältlich unter ATCC 39,038) ist.

11. Verfahren zur Gewinnung eines Segmentes für die kontrollierte Weitergabe in B. subtilis nach Anspruch 1 oder 2, gekennzeichnet durch die folgenden Schritte:

(a) Restriktionsenzym-Spaltung eines in einer B. subtilis-Spezies A natürlich vorkommenden Plasmids oder eines konstruierten rekombinanten B. subtilis-Plasmids, das bekanntermaßen kontrolliert weitergegeben wird;

(b) beliebige Clonierung der in Schritt (a) erhaltenen DNA-Fragmente in ein Plasmid, das sowohl in einer bakteriellen Spezies B als auch in in B. subtilis C replikationsfähig ist und einen Selektionsmarker trägt;

(c) Transformation einer Population von Organismen der Spezies B mit den im Schritt (b) hergestellten rekombinanten Plasmiden;

(d) Kultivierung der im Schritt (c) transformierten Organismen der Spezies B unter einem Selektionsdruck, der dem Selektionsmarker auf den im Schritt (b) hergestellten Plasmiden entspricht;

(e) Isolierung der Plasmide von den im Schritt (d) vermehrten Organismen der Spezies B;

(f) Transformation einer Population von B. subtilis mit den in Schritt (e) isolierten Plasmiden;

(g) Kultivierung der Population von transformierten B. subtilis unter dem dem Selektionsmarker auf den Plasmiden entsprechenden Selektionsdruck, wobei die Kultivierung ausreichend viele Verdoppelungszeiten umfaßt, um im wesentlichen alle Organismen zu entfernen, die nicht mit den Plasmiden im Schritt (f) transformiert worden sind;

(h) Kultivierung der nach Schritt (g) verbliebenen Population von Organismen in Abwesenheit des dem Selektionsmarker entsprechenden Selektionsdrucks, wobei die Kultivierung ausreichend viele Verdoppelungszeiten umfaßt, um den Anteil der Organismen in der Population, die jene im Schritt (b) hergestellten Plasmide für die kontrollierte Weitergabe tragen, auf mindestens 0,5 zu erhöhen;

(i) Isolierung der Plasmide aus der nach Schritt (h) erhaltenen Population von Organismen;

(j) Spaltung der im Schritt (i) erhaltenen Plasmide mit einem Restriktionsenzym; und

(k) Isolierung des Segments für die kontrollierte Weitergabe in gram-positiven Bakterien auf den im Schritt (j) erhaltenen Fragmenten.

12. Verfahren zur Herstellung von konstruierten rekombinanten Plasmiden, die während der Zellteilungen von B. subtilis kontrolliert weitergegeben werden, dadurch gekenzeichnet, daß B. subtilis Weitergabe-Segmente oder Fragmente nach Anspruch 1 oder 2 in rekombinante Plasmide inseriert werden.

13. Verfahren nach Anspruch 11 oder 12, wobei das konstruierte rekombinante Plasmid, in welches die ß. subtilis Weitergabe-Segmente oder Fragmente inseriert sind, das Plasmid pOG1196 (erhältlich unter ATCC 31,776) ist.

14. Verfahren zur Gewinning einer Population von B. subtilis, die als Teil ihres Genotyps phenotypisch exprimierbare Plasmide mit kontrollierter weitergabe trägt, gekennzeichnet, durch die folgenden Schritte:

Transformation von B. subtilis mit einem Plasmid nach Anspruch 3 oder 4, anschließend, falls geeignet, oder wenn bereits eine Population von B. subtilis mit rekombinanten Plasmiden vorliegt, die einen Selektionsmarker tragen, Erhöhung des Anteils der Organismen, in denen die Plasmide kontrolliert weitergegeben werden, in dem die Organismen in Abwesenheit des dem Selektionsdruck entsprechenden Selektionsmarkers kultiviert werden.

15. Verfahren nach Anspruch 14, wobei der Selektionsmarker eine Antibiotika-Resistenz aufweist.

16. Verfahren nach Anspruch 15, wobei das Antibiotikum Chloramphenicol ist.

17. Verfahren nach einem der Ansprüche 14 bis 16, wobei der B. subtilis-Stamm der Stamm BD224 (erhältlich unter ATCC 39,038) ist.

18. Verfahren nach einem der Ansprüche 14 bis 17, wobei das Wachstum bis zu einer ausreichenden Anzahl von Verdoppelungszeiten fortgesetzt wird, um den Anteil der Organismen, die Plasmide mit kontrollierter Weitergabe tragen, auf mehr als etwa 0,90 zu erhöhen.

19. Verfahren zur Herstellung eines Protein-Produkts, gekennzeichnet, durch die folgenden Schritte:

Kultivierung eines Organismus nach einem der Ansprüche 5 bis 7 oder einer Population von B. subtilis nach Anspruch 14 und Erhalten nach dem Verfahren nach Anspruch 14, wobei das konstruierte rekombinante Plasmid für die kontrollierte Weitergabe das genetische Material für die Herstellung des Protein-Produkts trägt, und Ermöglichung für den Organismus seinen Genotyp einschließlich des Plasmids phenotypisch zu exprimieren.

**Revendications**

1. Segment à capacité de répartition efficace dans *B. subtilis,* qui confère, une fois intégré dans un plasmide recombinant susceptible de réplication dans *B. subtilis,* une capacité de répartition audit plasmide construit recombinant dans *B. subtilis,* et qui peut être obtenu à partir du plasmide construit recombinant pOG2381 de *B. subtilis* (pouvant être obtenu à partir de ATCC 39 038), sous la forme de fragment *MboI* ou *Sau3A* d'environ 1 200 paires de bases ou de fragments *Hind*III—*Hae*III d'environ 350 paires de bases ou de fragments *Hae*III—*Pst*I d'environ 260 paires de bases, ou de fragments d'ADN ayant une homologie avec au moins une partie de l'un quelconque des fragments précités, et dans lequel les fragments homologues confèrent une capacité de répartition à des plasmides recombinants dans *B. subtilis.*

2. Segment à capacité de répartition comprenant au moins une partie de la séquence nucléotidique de la figure 6 ci-annexée, ou un fragment d'ADN ayant une homologie avec celle-ci, et dans lequel ledit segment ou fragment est capable de conférer une capacité de répartition à des plasmides recombinants dans *B. subtilis.*

3. Plasmide recombinant de *B. subtilis* à capacité de répartition, qui, une fois transformé dans *B. subtilis,* est capable de répartition dans celui-ci, et comprend un segment ou fragment de répartition selon la revendication 1 ou 2.

4. Plasmide pOG2381 (pouvant être obtenu à partir d'AtCC 39 038).

5. *B. subtilis* transformé par un plasmide recombinant selon la revendication 3 ou 4.

6. *B. subtilis* selon la revendication 5, qui est un organisme *Bacillus subtilis* transformé (recE$^-$).

7. *B. subtilis* selon la revendication 6, qui est la souche *Bacillus subtilis* BD224 (pouvant être obtenue à partir d'ATCC 39 038).

8. Procédé pour l'obtention de segments de répartition efficaces dans *B. subtilis,* lequel procédé comprend la ligature de fragments d'ADN tels que définis dans la revendication 1 ou la revendication 2, en un site d'enzyme de restriction compatible produit par digestion enzymatique d'un plasmide capable de transformer un hôte de type *B. subtilis* pour la production de plasmides construits incorporant lesdits fragments d'ADN, l'utilisation des plasmides construits résultant pour transformer les cellules de l'hôte, la croissance des cellules hôtes après transformation et la sélection de transformants, et, si on le désire, la digestion enzymatique des plasmides résultants capables de répartition, et l'isolement des segments de répartition de *B. subtilis* à partir du produit de digestion.

9. Procédé selon la revendication 8, dans lequel le plasmide capable de transformer un hôte de type *B. subtilis* est le plasmide pOG1196 (pouvant être obtenu à partir d'ATCC 31 776).

10. Procédé selon la revendication 8 ou la revendication 9, dans lequel la souche de *Bacillus subtilis* est *Bacillus subtilis* souche BD244 (pouvant être obtenue à partir d'ATCC 39 038).

11. Procédé pour l'obtention d'un segment de répartition efficace de *B. subtilis,* tel que défini dans la revendication 1 ou la revendication 2, lequel comprend

(a) la digestion par une enzyme de restriction d'un plasmide existant à l'état naturel dans un type A de *B. subtilis* ou d'un plasmide de *B. subtilis* construit recombinant, connu comme étant capable de répartition;

(b) le clonage au hasard des fragments d'ADN résultants, obtenus dans l'étape (a), dans un plasmide capable de réplication dans une espèce bactérienne B ainsi que dans *B. subtilis* C, et portant un marquer sélectionnable;

(c) la transformation d'une population d'organismes de l'espèce B par les plasmides recombinants produits dans l'étape (b);

(d) la culture d'organismes de l'espèce B transformés comme dans l'étape (c), sous une pression de sélection correspondant au marquer sélectionnable sur le plasmide produit dans l'étape (b);

(e) la récolte des plasmides à partir des organismes de l'espèce B développés comme dans l'étape (d);

(f) la transformation d'une population de *B. subtilis* au moyen des plasmides récoltés comme dans l'étape (e);

(g) la culture de la population de *B. subtilis* transformés, sous la pression de sélection à laquelle correspond le marquer sélectionnable sur les plasmides, pendant une durée correspondant à un nombre de temps de doublement suffisant pour éliminer pratiquement tous les organismes non transformés par les plasmides dans l'étape (f);

(h) la culture de la population d'organismes restant après l'étant (g), en l'absence de la pression de sélection correspondant au marquer sélectionnable, pendant une durée correspondant à un nombre de temps de doublement suffisant pour accroître d'un facteur d'au moins 0,5 la fraction d'organismes dans la population qui portent des plasmides de répartition produits dans l'étape (b);

(i) l'isolement des plasmides à partir de la population d'organismes résultants après l'étape (h);

(j) la digestion par une enzyme de restriction des plasmides obtenus dans l'étape (i); et

(k) l'isolement du segment de répartition Gram positif à partir des fragments obtenus dans l'étape (j).

12. Procédé pour la création de plasmides recombinants construits qui seront capables de répartition lors de divisions de cellules de *B. subtilis*, comprenant la ligature de segments ou fragments de répartition de *B. subtilis* tels que définis dans la revendication 1 ou la revendication 2, dans des plasmides recombinants.

13. Procédé selon la revendication 11 ou la revendication 12, dans lequel le plasmide recombinant construit, dans lequel les segments ou fragments de répartition de *B. subtilis* sont ligaturés, est le plasmide pOG1196 pouvant être obtenu à partir d'ATCC 31776).

14. Procédé pour l'obtention d'une population de *B. subtilis* comportant, en tant que partie de leur génotyoe, des plasmides capables de répartition et exprimables phénotypiquement, lequel procédé comprend la transformation de *B. subtilis* par un plasmide selon la revendication 3 ou la revendication 4, suivie par (si cela est approprié), ou remplacée par l'accroissement, dans une population de *B. subtilis* hébergeant des plasmides recombinants qui portent un marquer sélectionnable, de la fraction des organismes dans lesquels les plasmides sont capables de répartition, par culture des organismes en l'absence de la pression de sélection correspondant au marquer sélectionnable.

15. Procédé selon la revendication 14, dans lequel le marquer sélectionnable est la résistance à un antibiotique.

16. Procédé selon la revendication 15, dans lequel l'antibiotique est le chloramphénicol.

17. Procédé selon l'une quelconque des revendications 14 à 16, dans lequel la souche de *B. subtilis* est la souche *Bacillus subtilis* BD224 (pouvant être obtenue à partir d'ATCC 39 038).

18. Procédé selon l'une quelconque des revendications 14 à 17, dans lequel on poursuit la croissance pendant une durée correspondant à un nombre de temps de doublement suffisant pour accroître à plus de 0,90 environ la fraction d'organismes porteurs de plasmides capables de répartition.

19. Procédé pour la préparation d'un produit protéique, comprenant la culture d'un organisme selon l'une quelconque des revendications 5 à 7, ou d'une population de *B. subtilis* telle que définie dans la revendication 14 et telle qu'obtenue par un procédé selon la revendication 14, dans lequel le plasmide construit recombinant, capable de répartition, porte du matériau génétique codé pour la production dudit produit protéique, et on permet à l'organisme d'exprimer phénotypiquement son génotype comprenant ledit plasmide.

FIG. 1

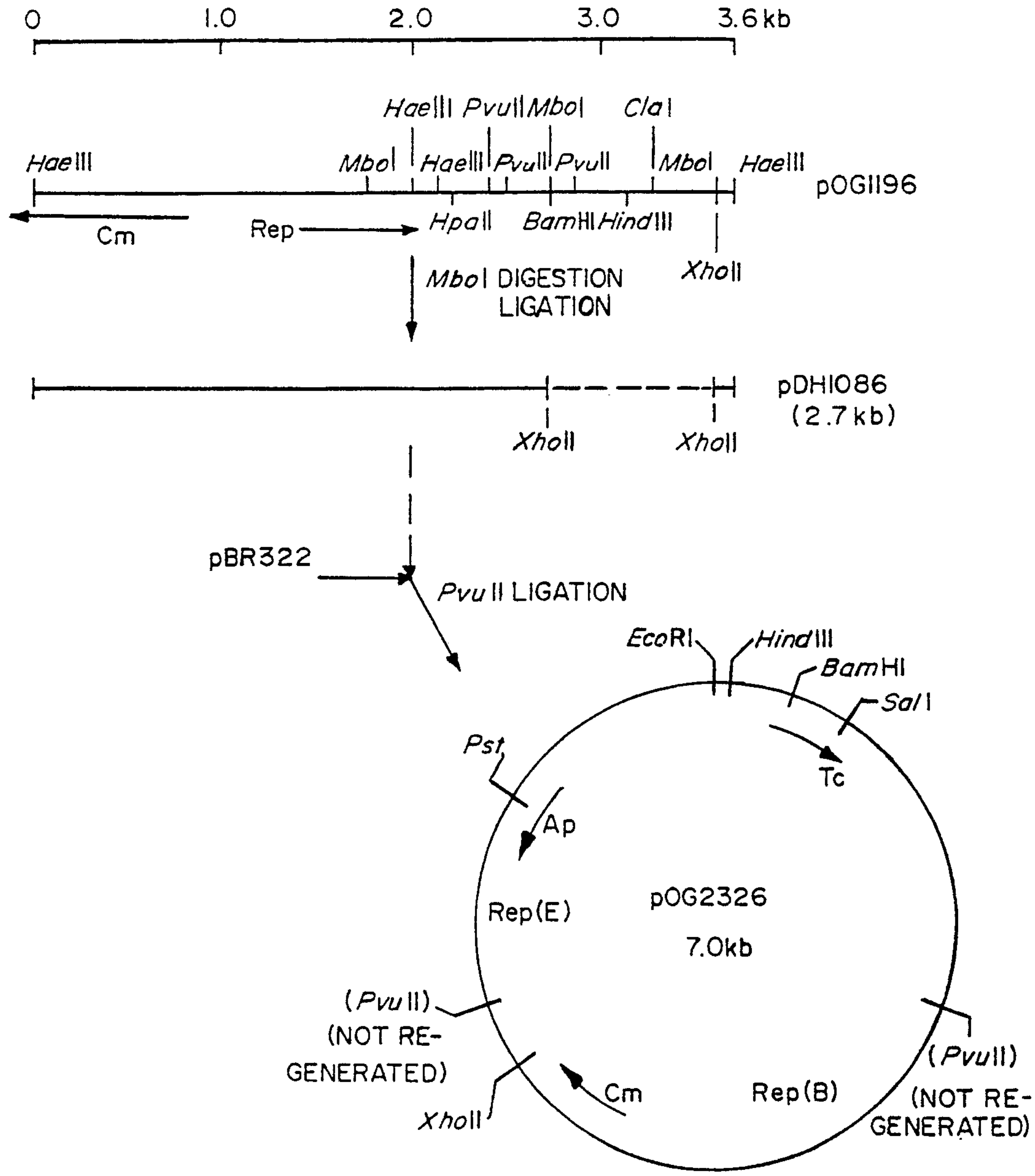
0
1.0
2.0
3.0
3.6 kb
HaeIII
PvuII
MboI
ClaI
HaeIII
MboI
HaeIII
PvuII
PvuII
MboI
HaeIII
pOG1196
Cm
Rep
HpaII
BamHI
HindIII
XhoII
MboI DIGESTION
LIGATION
pDH1086
(2.7 kb)
XhoII
XhoII
pBR322
PvuII LIGATION
EcoRI
HindIII
BamHI
SalI
Tc
Pst
Ap
Rep(E)
pOG2326
7.0kb
(PvuII)
(NOT RE-
GENERATED)
XhoII
Cm
Rep(B)
(PvuII)
(NOT RE-
GENERATED)

FIG. 2

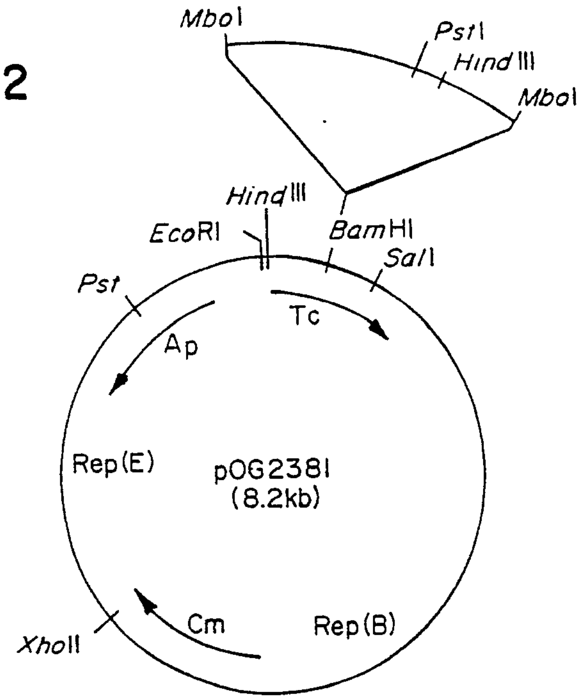
MboI
PstI
HindIII
MboI
HindIII
EcoRI
BamHI
SalI
Pst
Tc
Ap
Rep(E)
pOG2381
(8.2kb)
XhoII
Cm
Rep(B)

FIG. 3

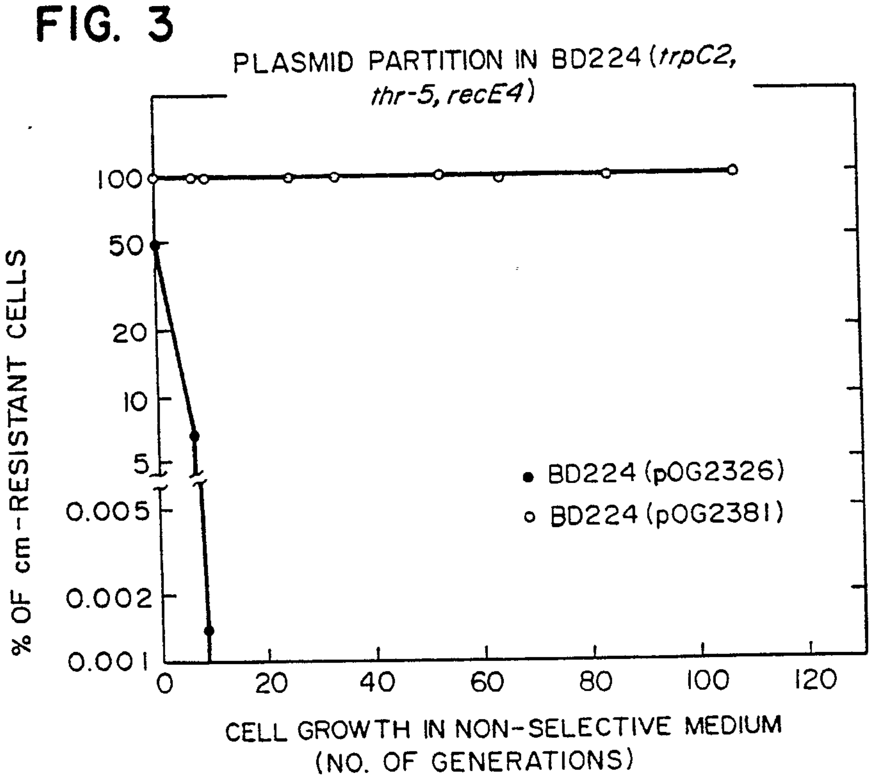
PLASMID PARTITION IN BD224 (trpC2, thr-5, recE4)
% OF cm-RESISTANT CELLS
100
50
20
10
5
0.005
0.002
0.001
0
20
40
60
80
100
120
• BD224 (pOG2326)
o BD224 (pOG2381)
CELL GROWTH IN NON-SELECTIVE MEDIUM
(NO. OF GENERATIONS)

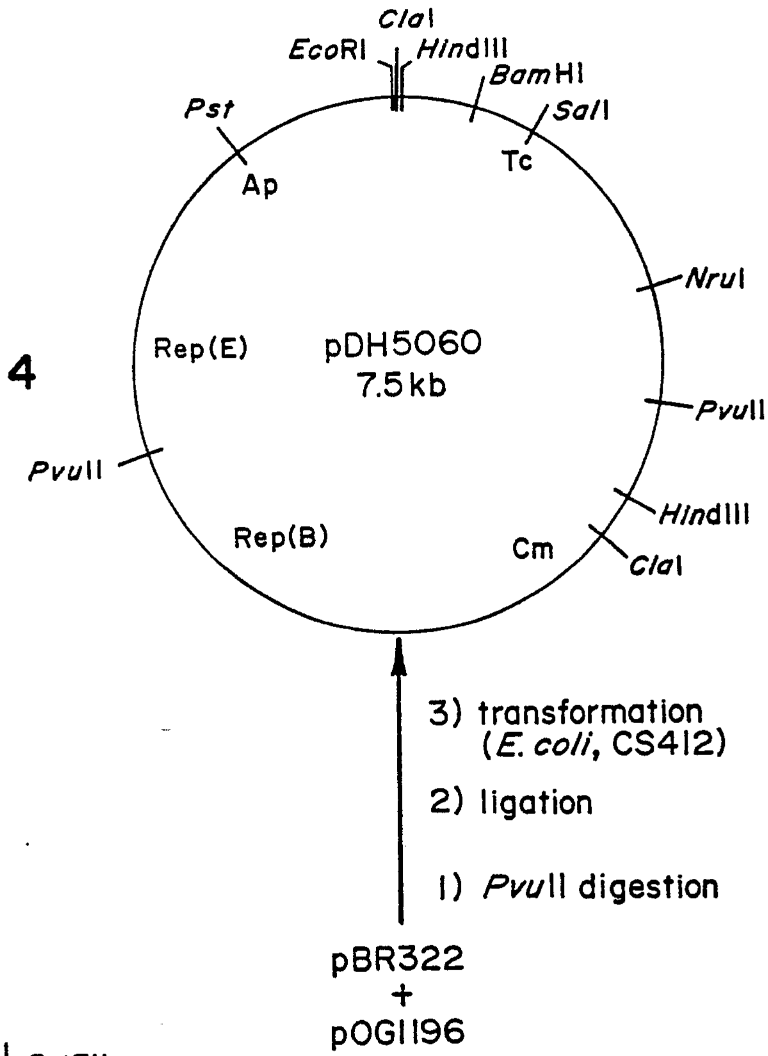
ClaI
EcoRI
HindIII
BamHI
SalI
Pst
Ap
Tc
NruI
Rep(E)
pDH5060
7.5kb
PvuII
PvuII
HindIII
Rep(B)
Cm
ClaI
3) transformation
(E. coli, CS412)
2) ligation
1) PvuII digestion
pBR322
+
pOGI196

FIG. 4

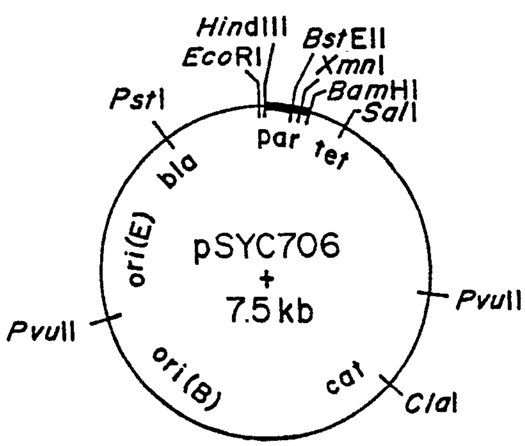
HindIII
EcoRI
BstEII
XmnI
BamHI
SalI
PstI
par
tet
bla
ori(E)
pSYC706
+
7.5kb
PvuII
PvuII
ori(B)
cat
ClaI

FIG. 5

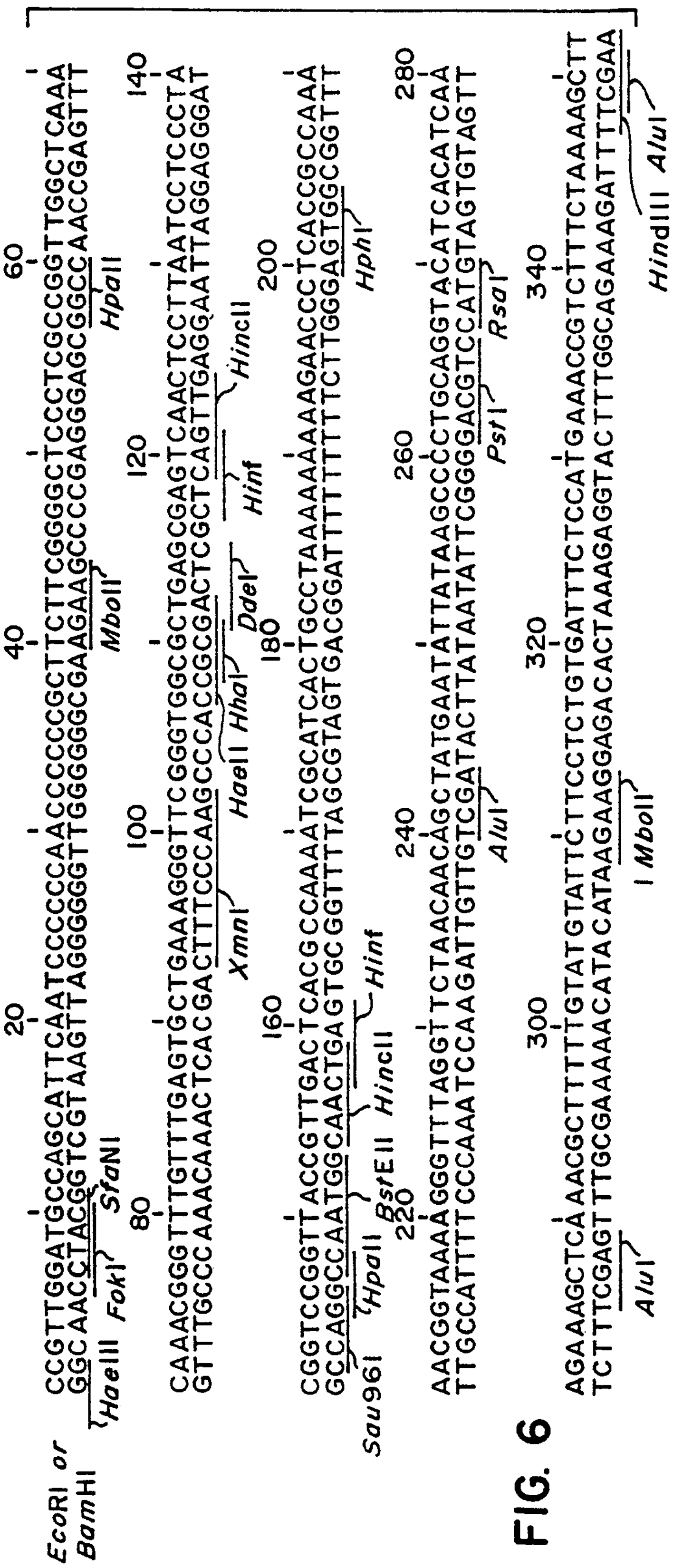

EcoRI or BamHI
HaeIII FokI SfaNI MboII HpaII
XmnI HaeII HhaI DdeI Hinf HincII
Sau961 HpaII BstEII HincII Hinf HphI
AluI PstI RsaI
AluI I MboII HindIII AluI


FIG. 6

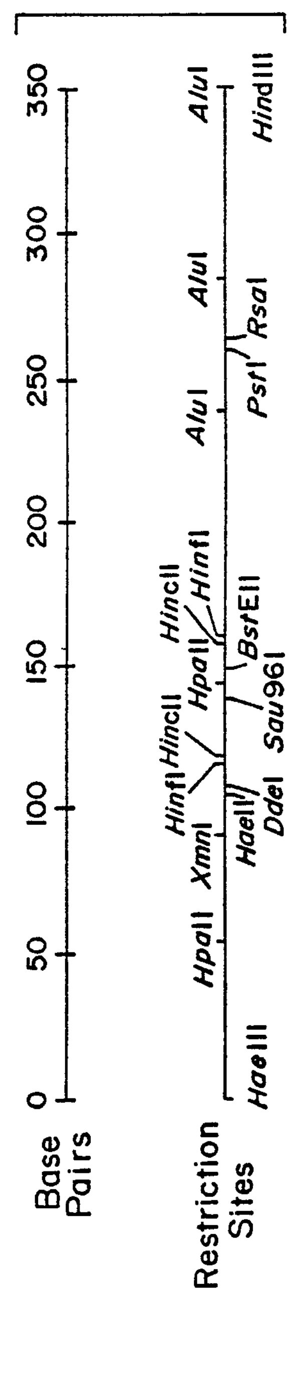

Base Pairs
0
50
100
150
200
250
300
350
Restriction Sites
HaeIII HpaII XmnI HinfI HincII HpaII HincII HinfI AluI AluI AluI
HaeII DdeI Sau961 BstEII PstI RsaI HindIII


FIG. 7